# EUROPÄISCHE PATENTSCHRIFT

(11) **EP 2 645 929 B1**
(45) Veröffentlichungstag und Bekanntmachung des Hinweises auf die Patenterteilung: **16.10.2019**
(21) Anmeldenummer: 11817510.8
(22) Anmeldetag: 05.09.2011
(51) Int. Cl.: A61B 5/107, G01B 5/06, F16C 11/04, F16C 11/12, F16C 17/00, F16C 11/10

(54) **VORRICHTUNG ZUR LÄNGENMESSUNG MIT KOPFANSCHLAG**
DEVICE FOR HEIGHT MEASUREMENT HAVING A HEADPIECE
DISPOSITIF DESTINÉ À MESURER LA TAILLE, POURVU D'UNE BUTÉE DE TÊTE

(30) Priorität: 30.11.2010 DE 102010054023
(43) Veröffentlichungstag der Anmeldung: 09.10.2013
(73) Patentinhaber: seca ag, 4153 Reinach BL 1 (CH)
(72) Erfinder: VON MAYDELL, Marc-Oliver, 22337 Hamburg (DE)
(74) Vertreter: Klickow & Wetzel PartGmbB
(86) Internationale Anmeldenummer: PCT/DE2011/001726
(87) Internationale Veröffentlichungsnummer: WO 2012/092910

(56) Entgegenhaltungen:
- DE-U1-202005 003 048
- US-A- 4 495 702
- US-A1- 2005 155 246

## Beschreibung

Die Erfindung betrifft eine Vorrichtung zur Längenmessung von Personen, die ein Vertikalelement und einen am Vertikalelement positionierbar angeordneten Kopfanschlag aufweist, der relativ zum Vertikalelement durch ein Drehgelenk verdrehbar angeordnet ist.

Derartige Vorrichtungen sind typischerweise als sogenannte Längenmessstäbe ausgebildet. Die Längenmessstäbe können unter Verwendung eines Fußes selbststehend sein. Ebenfalls können derartige Längenmessstäbe an einer Tragstruktur, beispielsweise an einer Wand oder einer Waage, befestigt werden.

Zur Vorgabe einer definierten Positionierung einer zu vermessenden Person und zur Unterstützung einer Erfassung des Messergebnisses wird ein Kopfanschlag verwendet. Der Kopfanschlag kann entlang eines Vertikalelementes der Vorrichtung verschiebbar sein oder durch bewegliche Segmente eines Vertikalelementes positioniert werden. Die DE 198 08 432 schreibt beispielsweise die Positionierung eines derartigen Kopfanschlages unter Verwendung von teleskopierbaren Segmenten des Vertikalelementes.

In einem Benutzungszustand erstreckt sich der Kopfanschlag im Wesentlichen rechtwinklig zum Vertikalelement. Zur Ermöglichung einer kompakten Verstauung der Vorrichtung kann der Kopfanschlag auch verschwenkbar zum Vertikalelement angeordnet werden. Bei einem Hoch- oder Herunterschwenken des Kopfanschlages erfolgt jedoch hierbei typischerweise eine Behinderung der zu vermessenden Person.

Aus der US 2005/155246 A1 ist bereits eine Vorrichtung zur Längenmessung von Personen bekannt, die ein Vertikalelement und einen am Vertikalelement positionierbar angeordneten Kopfanschlag aufweist. Der Kopfanschlag ist relativ zum Vertikalelement durch ein Drehelement verdrehbar angeordnet.

Aus der DE 20 2005 003 048 U1 ist eine Vorrichtung bekannt, bei der ein Teilelement aus einer vertikalen Lage in eine horizontale Lage verbracht werden kann.

Aufgabe der vorliegenden Erfindung ist es, eine Vorrichtung der einleitend genannten Art derart zur konstruieren, dass eine verschwenkbare Anordnung des Vertikalelementes ohne eine Beeinträchtigung des Benutzungskomforts erreicht wird. Diese Aufgabe wird erfindungsgemäß dadurch gelöst, dass eine Drehachse des Drehgelenkes schräg zu einer Längsachse des Vertikalelementes angeordnet ist, dass die Drehachse geneigt zu einer horizontalen Richtung verläuft, dass sich der Kopfanschlag in einer Arbeitspositionierung mit einer Längsachse im Wesentlichen quer zu einer Längsachse des Vertikalelementes erstreckt und dass für mindestens eine Positionierung des Kopfanschlages relativ zum Vertikalelement eine Rastung verwendet ist.

Durch die Anordnung der Drehachse des Drehgelenkes schräg zur Längsachse des Vertikalelementes ist es möglich, bei der Durchführung der Schwenkbewegung eine Bewegungskomponente des Kopfanschlages in Richtung auf den Benutzer zu vermeiden Weiter auf Seite 3 der Ursprungsunterlagen beziehungsweise auf einen räumlich geringen Bereich zu begrenzen. Im Vergleich zur Verwendung einer horizontal verlaufenden Drehachse ist es somit nicht erforderlich, dass der Benutzer dem Kopfanschlag bei der Durchführung der Schwenkbewegung ausweichen muss.

Eine geschwungene Bewegungsbahn für den Kopfanschlag kann insbesondere dadurch vorgegeben werden, dass die Drehachse geneigt zu einer horizontalen Richtung verläuft.

Eine typische Ausführungsform wird dadurch bereitgestellt, dass die Drehachse einen Neigungswinkel von etwa 45 Grad relativ zur horizontalen Richtung aufweist.

Eine einfache und robuste Gestaltung wird dadurch erreicht, dass das Drehgelenk einen Zapfen sowie zwei Drehflächen aufweist.

Eine vorteilhafte konstruktive Gestaltung besteht darin, dass die eine Drehfläche mit dem Vertikalelement und die andere Drehfläche mit dem Kopfanschlag verbunden ist.

Eine zweckmäßige räumliche Orientierung besteht darin, dass die Drehflächen im Wesentlichen quer zur Drehachse verlaufen.

Zur Unterstützung eines raumsparenden Transportes der Vorrichtung wird vorgeschlagen, dass sich der Kopfanschlag mit einer Längsachse in einer Grundpositionierung im Wesentlichen parallel zu einer Längsachse des Vertikalelementes erstreckt.

Eine genaue und reproduzierbare Längenmessung wird dadurch unterstützt, dass sich der Kopfanschlag in einer Arbeitspositionierung mit einer Längsachse im Wesentlichen quer zu einer Längsachse des Vertikalelementes erstreckt.

Eine vorgebbare Positionierung wird dadurch unterstützt, dass für mindestens eine Positionierung des Kopfanschlages relativ zum Vertikalelement eine Rastung verwendet ist.

Zu einer kompakten Gestaltung trägt es ebenfalls bei, dass das Vertikalelement und/oder der Kopfanschlag teleskopierbar ausgebildet ist.

In den Zeichnungen sind Ausführungsbeispiele der Erfindung schematisch dargestellt. Es zeigen:
- Figur 1: eine Vorderansicht eines Längenmessstabes mit verschwenkbarem Kopfanschlag,
- Figur 2: eine Seitenansicht gemäß Blickrichtung II in Fig. 1 mit gestrichelt eingezeichneter hochgeschwenkter Positionierung des Kopfanschlages,
- Figur 3: ein Ausführungsbeispiel für eine Realisierung des Schwenkgelenkes und
- Figur 4: die Anordnung gemäß Fig. 3 in einem hochgeschwenkten Zustand des Kopfanschlages.

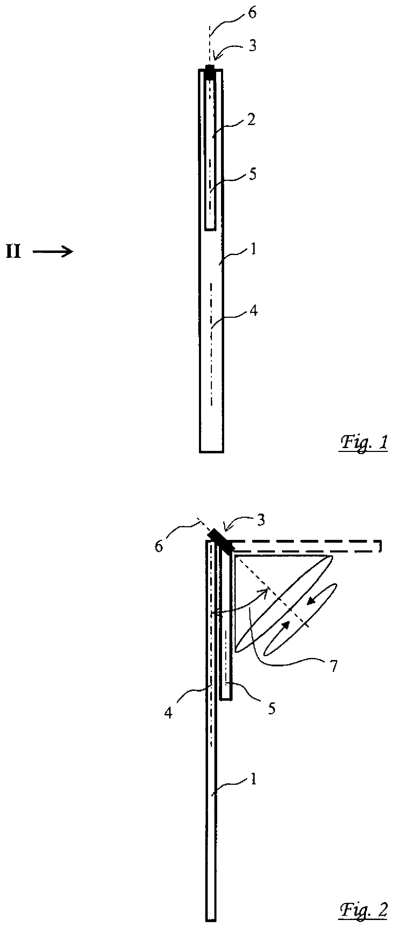

Gemäß der Ausführungsform in Fig. 1 weist die Vorrichtung zur Längenmessung ein Vertikalelement (1) auf, das beweglich mit einem Kopfanschlag (2) verbunden ist. Die Verbindung des Kopfanschlages (2) mit dem Vertikalelement (1) erfolgt unter Verwendung eines Schwenkgelenkes (3). Fig. 1 zeigt den Kopfanschlag (2) in einer heruntergeschwenkten Positionierung.

Fig. 2 veranschaulicht die Konstruktion gemäß Fig. 1 in einer Seitenansicht. Es ist zu erkennen, dass sich in dieser heruntergeschwenkten Positionierung des Kopfanschlages (2) Längsachsen (4, 5) des Vertikalelementes (1) einerseits und des Kopfanschlags (2) andererseits im wesentlichen parallel zueinander erstrecken. Hierdurch wird eine kompakte Verstauung unterstützt. Ebenfalls ist zu erkennen, dass sich eine Drehachse (6) des Schwenkgelenkes (3) schräg zur Längsachse (4) des Vertikalelementes (1) erstreckt. Beim dargestellten Ausführungsbeispiel beträgt ein Neigungswinkel (7) der Drehachse (6) relativ zur Längsachse (4) etwa 45 Grad. Ausgehend vom Vertikalelement (1) verläuft die Drehachse (6) mit einem Gefälle.

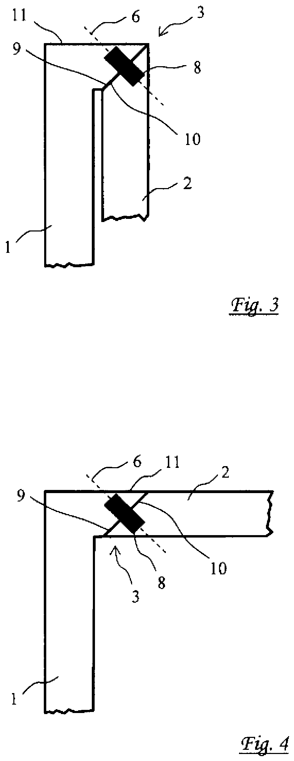

Fig. 3 veranschaulicht die genauere Konstruktion des Schwenkgelenkes (3). Das Schwenkgelenk (3) besteht hierbei aus einem Zapfen (8) und zwei Drehflächen (9, 10), die sich einander zugewandt erstrecken. Die Drehfläche (9) ist hierbei mit dem Vertikalelement (1) und die Drehfläche (10) mit dem Kopfanschlag (2) verbunden.

Beim dargestellten Ausführungsbeispiel sind die Drehflächen (9) und (10) eben gestaltet und verlaufen quer zur Drehachse (6). Der Zapfen (8) verläuft in Richtung der Drehachse (6).

Gemäß dem Ausführungsbeispiel in Fig. 3 weist das Vertikalelement (1) einen seitlichen Vorsprung (11) auf, an dem die Drehfläche (9) angebracht ist.

Fig. 4 zeigt die Anordnung gemäß Fig. 3 nach einem Hochschwenken des Kopfanschlages (2). Dies erfolgt durch ein Verdrehen um die Drehachse (6) herum. Bei einer Durchführung der Drehbewegung ausgehend vom Betriebszustand in Fig. 3 führt der Kopfanschlag (2) zunächst eine seitliche Bewegung durch und wird dann entlang einer geschwungenen Bewegungsbahn in den Betriebszustand gemäß Fig. 4 versetzt. Die Positionierung gemäß Fig. 3 und/oder gemäß Fig. 4 kann durch Rastungen vorgegeben beziehungsweise gesichert sein.

Die vorstehend beschriebene Vorrichtung zur Längenmessung kann als Einzelgerät oder in Kombination mit einer Waage zur Messung des Gewichtes einer Person verwendet werden. Eine Erfassung der Längeninformation kann visuell durch Ablesen einer Messskala oder messtechnisch unter Verwendung geeigneter Sensoren erfolgen. Die gemessenen Längeninformationen können gegebenenfalls zu Auswertungsgeräten übertragen werden.

Das Schwenkgelenk (3) kann beispielsweise fest mit einem teleskopierbaren Vertikalelement (1) verbunden sein. Möglich ist aber auch eine bewegliche Anordnung auf dem Vertikalelement (1). Für die Verwendung des erfindungsgemäßen Schwenkgelenkes ist es nicht erforderlich, dass die zu messende Person bei einem Hochschwenken des Kopfanschlages den Bereich der Längenmesseinrichtung verlässt. Der Kopfanschlag führt vielmehr bei der Hochschwenkbewegung eine kreissegmentartige Bewegung um den Kopf des Benutzers herum durch.

Alternativ zur dargestellten ebenen Ausbildung der Drehflächen (9, 10) ist es beispielsweise auch möglich, ineinander eingreifende ringartige Profilierungen oder kalottenartig gebogene Drehflächen (9, 10) zu verwenden. Wesentlich bei allen Ausführungsformen ist die ausgehend vom Vertikalelement (1) geneigt nach unten verlaufende Drehachse (6).

## Patentansprüche

1. Vorrichtung zur Längenmessung von Personen, die ein Vertikalelement (1) und einen am Vertikalelement (1) positionierbar angeordneten Kopfanschlag (2) aufweist, der relativ zum Vertikalelement (1) durch ein Drehgelenk (3) verdrehbar angeordnet ist und sich der Kopfanschlag (2) in einer Arbeitspositionierung mit einer Längsachse (5) im Wesentlichen quer zu einer Längsachse (4) des Vertikalelementes (1) erstreckt, **dadurch gekennzeichnet, dass** eine Drehachse (6) des Drehgelenkes (3) schräg zu einer Längsachse (4) des Vertikalelementes (1) angeordnet ist, dass die Drehachse (6) geneigt zu einer horizontalen Richtung verläuft und dass für mindestens eine Positionierung des Kopfanschlages (2) relativ zum Vertikalelement (1) eine Rastung verwendet ist.

2. Vorrichtung nach Anspruch 1, **dadurch gekennzeichnet, dass** die Drehachse (6) einen Neigungswinkel von etwa 45 Grad relativ zur horizontalen Richtung aufweist.

3. Vorrichtung nach einem der Ansprüche 1 oder 2, **dadurch gekennzeichnet, dass** das Drehgelenk (3) einen Zapfen (8) sowie zwei Drehflächen (9,10) aufweist.

4. Vorrichtung nach einem der Ansprüche 1 bis 3, **dadurch gekennzeichnet, dass** die Drehfläche (9) mit dem Vertikalelement (1) und die Drehfläche (10) mit dem Kopfanschlag (6) verbunden ist.

5. Vorrichtung nach einem der Ansprüche 1 bis 4, **dadurch gekennzeichnet, dass** die Drehflächen (9,10) im Wesentlichen quer zur Drehachse (6) verlaufen.

6. Vorrichtung nach einem der Ansprüche 1 bis 5, **dadurch gekennzeichnet, dass** für mindestens eine Positionierung des Kopfanschlages (2) relativ zum Vertikalelement (1) eine Rastung verwendet ist.

7. Vorrichtung nach einem der Ansprüche 1 bis 6, **dadurch gekennzeichnet, dass** für mindestens eine Positionierung des Kopfanschlages (2) relativ zum Vertikalelement (1) eine Arretierung verwendet ist.

8. Vorrichtung nach einem der Ansprüche 1 bis 7, **dadurch gekennzeichnet, dass** das Vertikalelement (1) teleskopierbar ausgebildet ist.

9. Vorrichtung nach einem der Ansprüche 1 bis 8, **dadurch gekennzeichnet, dass** der Kopfanschlag (6) entlang des Vertikalelementes (1) verschieblich angeordnet ist.

## Claims

1. Device for measuring the height of people, which has a vertical element (1) and a headpiece (2) arranged in a positionable manner on the vertical element (1), said headpiece (2) being arranged in a twistable manner relative to the vertical element (1) by way of a pivot joint (3), and the headpiece (2) extends, in a working position, with a longitudinal axis (5) substantially transverse to a longitudinal axis (4) of the vertical element (1), **characterized in that** an axis of rotation (6) of the pivot joint (3) is arranged obliquely with respect to a longitudinal axis (4) of the vertical element (1), **in that** the axis of rotation (6) extends in inclined manner with respect to a horizontal direction, and **in that** a catch is used for at least one position of the headpiece (2) relative to the vertical element (1).

2. Device according to Claim 1, **characterized in that** the axis of rotation (6) has an inclination angle of about 45 degrees relative to the horizontal direction.

3. Device according to either of Claims 1 and 2, **characterized in that** the pivot joint (3) has a peg (8) and two rotational surfaces (9, 10).

4. Device according to one of Claims 1 to 3, **characterized in that** the rotational surface (9) is joined to the vertical element (1) and the rotational surface (10) is joined to the headpiece (6) .

5. Device according to one of Claims 1 to 4, **characterized in that** the rotational surfaces (9, 10) extend substantially transversely to the axis of rotation (6).

6. Device according to one of Claims 1 to 5, **characterized in that** a catch is used for at least one position of the headpiece (2) relative to the vertical element (1).

7. Device according to one of Claims 1 to 6, **characterized in that** a locking mechanism is used for at least one position of the headpiece (2) relative to the vertical element (1).

8. Device according to one of Claims 1 to 7, **characterized in that** the vertical element (1) is formed in a telescopic manner.

9. Device according to one of Claims 1 to 8, **characterized in that** the headpiece (6) is arranged so as to be displaceable along the vertical element (1) .

## Revendications

1. Dispositif destiné à mesurer la taille de personnes, qui présente un élément vertical (1) et une butée de tête (2) disposée de manière à pouvoir être positionnée sur l'élément vertical (1), laquelle est disposée de manière à pouvoir tourner par rapport à l'élément vertical (1) par le biais d'une articulation pivotante (3), et la butée de tête (2) s'étend dans une position de travail avec un axe longitudinal (5) essentiellement transversalement à un axe longitudinal (4) de l'élément vertical (1), **caractérisé en ce qu'**un axe de rotation (6) de l'articulation pivotante (3) est disposé obliquement par rapport à un axe longitudinal (4) de l'élément vertical (1), **en ce que** l'axe de rotation (6) s'étend de manière inclinée par rapport à une direction horizontale et **en ce que** pour au moins une position de la butée de tête (2) par rapport à l'élément vertical (1), on utilise un crantage.

2. Dispositif selon la revendication 1, **caractérisé en ce que** l'axe de rotation (6) présente un angle d'inclinaison d'environ 45° par rapport à la direction horizontale.

3. Dispositif selon l'une quelconque des revendications 1 ou 2, **caractérisé en ce que** l'articulation pivotante (3) présente un tourillon (8) ainsi que deux surfaces de pivotement (9, 10).

4. Dispositif selon l'une quelconque des revendications 1 à 3, **caractérisé en ce que** la surface de pivotement (9) est connectée à l'élément vertical (1) et la surface de pivotement (10) est connectée à la butée de tête (6).

5. Dispositif selon l'une quelconque des revendications 1 à 4, **caractérisé en ce que** les surfaces de pivotement (9, 10) s'étendent essentiellement transversalement à l'axe de rotation (6).

6. Dispositif selon l'une quelconque des revendications 1 à 5, **caractérisé en ce que** pour au moins une position de la butée de tête (2) par rapport à l'élément vertical (1), on utilise un crantage.

7. Dispositif selon l'une quelconque des revendications 1 à 6, **caractérisé en ce que** pour au moins une position de la butée de tête (2) par rapport à l'élément vertical (1), on utilise un blocage.

8. Dispositif selon l'une quelconque des revendications 1 à 7, **caractérisé en ce que** l'élément vertical (1) est réalisé de manière télescopique.

9. Dispositif selon l'une quelconque des revendications 1 à 8, **caractérisé en ce que** la butée de tête (6) est disposée de manière déplaçable le long de l'élément vertical (1).
